(19) Europäisches Patentamt
European Patent Office
Office européen des brevets

(11) **EP 3 669 879 B1**

(12) **FASCICULE DE BREVET EUROPEEN**

(45) Date de publication et mention
de la délivrance du brevet:
**28.02.2024 Bulletin 2024/09**

(21) Numéro de dépôt: **19213282.7**

(22) Date de dépôt: **20.12.2013**

(51) Classification Internationale des Brevets (IPC):
**A61K 31/7048** (2006.01)    **A61K 9/00** (2006.01)
**A61K 47/36** (2006.01)    **A61K 9/10** (2006.01)
**A61P 9/00** (2006.01)

(52) Classification Coopérative des Brevets (CPC):
**A61K 47/36; A61K 9/0095; A61K 31/7048;**
**A61P 9/00**

(54) **COMPOSITION PHARMACEUTIQUE SOUS LA FORME D'UNE SUSPENSION ORALE COMPRENANT UNE FRACTION FLAVONOÏQUE COMPRENANT DE LA DIOSMINE ET DE LA GOMME XANTHANE**

PHARMAZEUTISCHE ZUSAMMENSETZUNG IN FORM EINER SUSPENSION ZUR EINNAHME, DIE EINE DIOSMIN-HALTIGE FLAVONOID FRAKTION UND XANTHANGUMMI ENTHÄLT

PHARMACEUTICAL COMPOSITION IN THE FORM OF AN ORAL SUSPENSION INCLUDING A FLAVONOID FRACTION COMPRISING DIOSMIN AND XANTHAN GUM

(84) Etats contractants désignés:
**AL AT BE BG CH CY CZ DE DK EE ES FI FR GB GR HR HU IE IS IT LI LT LU LV MC MK MT NL NO PL PT RO RS SE SI SK SM TR**
Etats d'extension désignés:
**BA ME**

(30) Priorité: **21.12.2012 FR 1203579**

(43) Date de publication de la demande:
**24.06.2020 Bulletin 2020/26**

(62) Numéro(s) de document de la (des) demande(s) initiale(s) en application de l'article 76 CBE:
**13198744.8 / 2 745 839**

(73) Titulaire: **Les Laboratoires Servier**
**92284 Suresnes (FR)**

(72) Inventeurs:
- **MARSAS, Stéphanie**
  **45430 Checy (FR)**
- **PEAN, Jean-Manuel**
  **45000 Orléans (FR)**

(74) Mandataire: **Giudicelli, Cathy**
**Les Laboratoires Servier**
**Direction Brevets**
**35, rue de Verdun**
**92284 Suresnes Cedex (FR)**

(56) Documents cités:
EP-A1- 0 711 560    EP-A1- 0 835 653
WO-A1-2009/083759    FR-A1- 2 661 610
FR-A1- 2 781 156    FR-M- 6 967
US-A1- 2012 070 475

- **"Recordati OTC Proctolyn Integra Plus", Mintel /GNPD, 1 novembre 2012 (2012-11-01), pages 1-1, XP055058166, Extrait de l'Internet: URL:http://www.gnpd.com/sinatra/gnpd/searc h_results&search_id=rPvvIZ9LtI/&item_id=19 18260 [extrait le 2013-03-28] & "Recordati OTC Proctolyn Integra Plus", , 1 novembre 2012 (2012-11-01), pages 1-1, XP055058167, Extrait de l'Internet: URL:http://www.gnpd.com/sinatra/gnpd/searc h_results&search_id=rPvvIZ9LtI/&item_id=19 18260 [extrait le 2013-03-28]**
- **FRIESENECKER B ET AL: "Oral administration of purified micronized flavonoid fraction suppresses leukocyte adhesion in ischemia-reperfusion injury: In vivo observations in the hamster skin fold", INTERNATIONAL JOURNAL OF MICROCIRCULATION, CLINICAL AND EXPERIMENTAL, BASEL, CH, vol. 14, no. 1-2, 1 janvier 1994 (1994-01-01), pages 50-55, XP009168459, ISSN: 0167-6865**

- CHAUMEIL J C: "MICRONIZATION: A METHOD OF IMPROVING THE BIOAVAILABILITY OF POORLY SOLUBLE DRUGS", METHODS AND FINDINGS IN EXPERIMENTAL AND CLINICAL PHARMACOLOGY, PROUS SCIENCE, vol. 20, no. 3, 1 avril 1998 (1998-04-01), pages 211-215, XP009048140, ISSN: 0379-0355

**Description**

La présente invention concerne une suspension orale buvable en sachet fortement dosée comprenant de la diosmine et optionnellement d'autres dérivés flavonoïques et comme excipient de la gomme xanthane, ainsi que son utilisation dans le traitement de l'insuffisance veineuse.

De préférence, la présente invention concerne une suspension buvable en sachet fortement dosée d'une fraction flavonoïque et de gomme xanthane pour l'administration orale, ainsi que son utilisation dans le traitement de l'insuffisance veineuse.

La fraction flavonoïque est issue d'un extrait de rutacées. La fraction flavonoïque purifiée et micronisée utilisée dans l'invention contient de 87% à 93% de diosmine et d'autres flavonoïdes de manière concomitante. Ces autres flavonoïdes à hauteur environ de 10% comprennent de 2.5% à 5.0% d'hespéridine, de 0.9% à 2.8% d'isorhoifoline, de 0.9% à 2.8% de linarine et moins de 1 % de diosmétine. La taille des particules de la fraction flavonoïque micronisée est strictement inférieure à $5\mu m$, voire inférieure à $4\mu m$, $3\mu m$, $2\mu m$ et même $1.6\mu m$.

La fraction flavonoïque selon l'invention est administrée à des doses journalières allant de 1000mg à 3000mg afin de traiter les manifestations de l'insuffisance veineuse chronique des membres inférieurs. Du fait d'une importante métabolisation de la fraction flavonoïque dans le tractus gastro-intestinal, ce principe actif doit être fortement dosé à chacune de ses administrations. Par ailleurs, les traitements à base de fraction flavonoïque sont des traitements au long cours imposants une prise facile afin de favoriser l'observance du traitement de la part des patients. En conséquence, il est préférable de développer des formes galéniques permettant une prise facile pour les personnes âgées et sans addition d'eau pour les patients ayant une consommation hors foyer.

L'utilisation en thérapeutique de la fraction flavonoïque extrait de rutacées selon l'invention a été décrite dans le brevet EP 0 711 560. Ce brevet décrit une composition de granulés effervescents fortement dosé à 1000mg de fraction flavonoïque. Cependant ces granulés effervescents sont à disperser dans l'eau avant consommation.

Des solutions buvables contenant des flavonoïdes et de la gomme xanthane ont été décrites dans le brevet US 5,240,732. Ce brevet divulgue une solution dans laquelle les flavonoïdes ont été dissouts dans un alcool contrairement à la présente invention qui concerne une suspension aqueuse dans laquelle la fraction flavonoïque est dispersée de manière homogène.

Des suspensions buvables contenant des polyphénols et des stabilisants de la dispersion ont été décrites dans la demande de brevet US 2012/0070475. Les suspensions selon la demande US 2012/0070475 sont faiblement dosées en quercétine et utilisent exclusivement la gomme gellane en tant que stabilisant de la dispersion.

Le document « Recordati OTC Proctolyn Integra Plus » du 1er novembre 2012, (http://www.gnpd.com/sinatra/gnpd/search results&search id=rPvvIZ9Ltl/&i tem id=1918260) décrit une composition pharmaceutique sous la forme de poudre en sachet, comprenant des agents épaississants dont la gomme xanthane et de la diosmine.

FR 2 661 610 A1 décrit une composition pharmaceutique lyophilisée comprenant de la diosmine et de la gomme xanthane pouvant être administrée via un milieu aqueux ou bien directement dans la salive.

FR 2 781 156 A1 décrit des granulés à disperser dans l'eau comprenant de la diosmine et de la gomme xanthane.

Enfin, le document FR 6967M divulgue une suspension buvable, devant être agitée avant emploi, de diosmine comprenant 0,5% de gomme adragante.

L'objet de la présente invention est composition pharmaceutique sous la forme d'une suspension orale fortement dosée comprenant comme principe actif de la diosmine et optionnellement d'autres dérivés flavonoïques et comme excipient de la gomme xanthane.

De préférence, l'objet de la présente invention est une composition pharmaceutique sous la forme d'une suspension orale buvable en sachet fortement dosée comprenant comme principe actif une fraction flavonoïque et comme excipient de la gomme xanthane.

Le pourcentage de fraction flavonoïque dans la composition pharmaceutique est compris entre 7%m/m et 20%m/m.

La quantité de fraction flavonoïque dans la composition pharmaceutique est comprise entre 1000mg et 3000mg, dont 2000mg, 1500mg, 2500mg.

La suspension orale fortement dosée en principe actif de la présente invention doit avoir une viscosité adéquate pour permettre d'une part, à la suspension de s'écouler dans les machines de fabrication et hors du sachet lors de l'administration et pour permettre d'autre part, à la fraction flavonoïque de ne pas sédimenter dans le sachet et être stable au cours du temps.

Afin de répondre à la totalité de ces propriétés fonctionnelles, la suspension orale doit répondre à des propriétés rhéologiques spécifiques.

La viscosité de la suspension ne doit pas dépendre du temps pendant lequel elle subit des forces de cisaillement. Il est nécessaire que la suspension retrouve sa viscosité initiale après application d'une contrainte quelques soit sa durée d'application. La viscosité de la suspension doit donc être réversible sur la gamme 0.01 et $1000s^{-1}$ de cisaillement. Cette propriété rhéologique, i.e. la suspension retrouve entièrement sa viscosité pendant les temps de repos, est essentielle dans les étapes de pompage, préemballage, en passant par le transport et le stockage jusqu'à l'utilisation par le consommateur final de la suspension orale en sachet.

Par ailleurs, il apparait nécessaire que la viscosité de la suspension soit indépendante de la température sur l'intervalle 15°C-60°C, gamme de températures ad hoc à l'issue de la production et au cours des étapes de transport, traitement et emballage du produit final.

Les mesures de viscosité sont réalisées sur le rhéomètre Anton Paar ou sur le rhéomètre de Brookfield. Les résultats obtenus avec ces deux types de rhéomètres sont tout à fait comparables.

Les propriétés rhéologiques et la stabilité requises par la suspension orale selon l'invention sont obtenues à l'aide d'un épaississement de la composition pharmaceutique. L'épaississement de la composition pharmaceutique est obtenu grâce à l'utilisation d'un agent épaississant.

L'agent épaississant selon l'invention est la gomme xanthane. La gomme xanthane est un polysaccharide anionique de haut poids moléculaire constituée de D-glucoses et de D-mannoses comme hexoses dominants. En solution, les molécules de gomme xanthane s'associent entre elles pour former un réseau de molécules enchevêtrées.

La concentration de gomme xanthane dans la composition pharmaceutique selon l'invention est comprise entre 0.45% m/v et 0.55% m/v. La concentration de la gomme xanthane peut être de 0.50% m/v, 0.47%m/v, 0.53%m/v.

La quantité de gomme xanthane dans la composition pharmaceutique est comprise entre 0.30%m/m et 0.60%m/m.

La composition pharmaceutique sous la forme d'une suspension orale buvable en sachet fortement chargée en fraction flavonoïque selon l'invention est sans tensioactifs. Les tensioactifs sont généralement utilisés dans les suspensions orales afin d'améliorer la dispersion et la mouillabilité des particules en suspension.

Outre la fraction flavonoïque et la gomme xanthane, la composition pharmaceutique selon l'invention contient un ou plusieurs excipients pharmaceutiquement acceptables tels que des conservateurs, des arômes, des édulcorants ou des correcteurs de pH.

A titre d'exemple d'excipients, on peut citer :

pour les conservateurs : benzoate de sodium, chlorure de benzalkonium, parahydroxybenzoate de méthyle, parahydroxybenzoate de propyle ;
pour les arômes : arôme orange, arôme citron, arôme caramel mou, arôme vanille/citron ;
pour les édulcorants : maltitol, le sorbitol, l'aspartam, le xylitol, l'acésulfame de potassium, la saccharine sodique ;
pour les correcteurs de pH : acide citrique, acide ascorbique, acide tartrique.

La composition pharmaceutique selon l'invention est sous la forme d'une suspension buvable en sachet. Le sachet ou stick a de préférence un volume unitaire de 10ml contenant la dose quotidienne de fraction flavonoïque. Pour une meilleure observance du patient le volume unitaire du sachet ne doit pas être trop important, par exemple 10ml, 12ml, 15ml, 17ml au plus 20ml.

La présente invention concerne également l'utilisation des compositions pharmaceutiques selon l'invention dans le traitement de la maladie veineuse, plus particulièrement de l'insuffisance veineuse. Ces compositions pharmaceutiques sont utilisées comme veinotonique et vasculo-protecteur.

Les exemples ci-dessous illustrent l'invention de manière non limitative.

Exemple 1 : Composition pharmaceutique pour sachet contenant une suspension de 1g de flavonoïdes

| | |
|---|---|
| Benzoate de sodium | 0.015g/10ml |
| Acide citrique | 0.0125g/10ml |
| Arôme orange | 0.015g/10ml |
| Maltitol | 1.8g/10ml |
| Fraction flavonoïque | 1.0g/10ml |
| Gomme xanthane | 0.05g/10ml |
| Eau purifiée | qsp 10ml |

Préparation de la suspension de l'exemple 1 :

Pour environ 100 000 sachets :

700 litres d'eau purifiée et 1.5kg de benzoate de sodium sont soigneusement mélangés à 20°C jusqu'à complète dissolution. 1.25kg d'acide citrique et 1.5kg d'arôme orange sont ajoutés à la solution. 180kg de poudre de maltitol sont ajoutés à la solution et soigneusement mélangés. 100kg de la fraction flavonoïque sont très lentement ajoutés au mélange jusqu'à la formation d'une suspension homogène. 5kg de gomme xanthane est introduite très lentement directement dans la suspension. De l'eau purifiée est ajoutée qsp obtenir un volume final de 1100 litres.

Exemple 2 : Stabilité de la suspension orale de l'exemple 1 (Batch LP02)

La stabilité de la composition pharmaceutique de l'Exemple 1 selon l'invention en sachet a été testée dans différentes conditions de température et d'humidité.

Les sachets sont constitués d'un complexe multicouche (polyéthylène PET12/aluminium AL12 /complexe de polyéthylène extrudé PE50).

| | Sachet | | | |
|---|---|---|---|---|
| | Densité (Eur. Ph.) | | | |
| T | 25°C / 60% HR | 30°C / 65% HR | 30°C / 75% HR | 40°C / 75% HR |
| T0 | 1.11 | | | |
| T0 + 1 mois | 1.10 | 1.10 | 1.10 | 1.10 |
| T0 + 3 mois | 1.10 | 1.10 | 1.10 | 1.10 |

Le tableau ci-dessus relatif à l'évolution de la densité de la suspension dans le sachet montre que la densité est totalement stable même dans des conditions de température et d'humidité élevées (40°C/75%HR). Cette stabilité de la densité montre que la suspension ne déphase pas au cours du temps dans des conditions de température et d'humidité élevées.

| | Sachet | | | | | | | |
|---|---|---|---|---|---|---|---|---|
| | Taille des particules de la fraction flavonoïque | | | | | | | |
| T | 25°C / 60% HR | | 30°C / 65% HR | | 30°C / 75% HR | | 40°C / 75% HR | |
| T0 | | | | | d10 0.658 | | | |
| | | | | | d50 2.581 | | | |
| | | | | | d90 6.642 | | | |
| T0 + 1 mois | d10 | 0.723 | d10 | 0.741 | d10 | 0.739 | d10 | 0.760 |
| | d50 | 2.679 | d50 | 2.692 | d50 | 2.694 | d50 | 2.726 |
| | d90 | 6.969 | d90 | 6.675 | d90 | 6.698 | d90 | 6.685 |
| T0 + 3 mois | d10 | 0.726 | d10 | 0.746 | d10 | 0.751 | d10 | 0.798 |
| | d50 | 2.663 | d50 | 2.779 | d50 | 2.708 | d50 | 2.818 |
| | d90 | 6.670 | d90 | 6.862 | d90 | 6.690 | d90 | 6.739 |

La distribution de taille des particules de la fraction flavonoïque est exprimée en diamètre d10, d50 et d90 où

d10=d(v,0.1) correspond à la valeur à 10% de la courbe cumulée de la distribution granulométrique en volume;

d50=d(v,0.5) correspond à la valeur à 50% de la courbe cumulée de la distribution granulométrique en volume;

d90=d(v,0.9) correspond à la valeur à 90% de la courbe cumulée de la distribution granulométrique en volume.

Le tableau ci-dessus montre que la taille des particules de la fraction flavonoïque selon l'invention n'augmente pas au cours du temps dans des conditions de température et d'humidité élevées. Les particules de la fraction flavonoïque micronisée ne s'agglomèrent pas ni ne sédimentent dans la suspension orale selon l'invention.

| T | Sachet | | | |
|---|---|---|---|---|
| | Viscosité en CP | | | |
| | 25°C / 60% HR | 30°C / 65% HR | 30°C / 75% HR | 40°C / 75% HR |
| T0 | 321 | | | |
| T0 + 1 mois | 293 | 298 | 294 | 296 |
| T0 + 3 mois | 293 | 293 | 294 | 298 |

Le tableau ci-dessus relatif à la viscosité de la suspension dans le sachet montre que la viscosité est extrêmement stable au cours du temps dans des conditions de température et d'humidité élevées.

Enfin, un contrôle visuel de la suspension dans les différentes conditions de température et d'humidité après 1 mois et 3 mois d'évaluation montre une absence de grumeaux et de bulles dans les sachets.

Exemple 3 : Uniformité de teneur de la composition pharmaceutique selon l'exemple 1

L'essai est effectué sur 10 sachets. Le contenu de chaque sachet est dosé ; une solution de référence de diosmine est utilisée comme contrôle.

La teneur moyenne $X_m$ est exprimée de la façon suivante :

$$X_m = (\Sigma\ X_i)/10$$

La valeur d'acceptation (AV), exprimée en pourcentage de la valeur théorique, est donnée par la formule suivante :

$$AV = (M - X_m) + k \times s$$

où :

$X_m$ est la teneur moyenne, exprimée en pourcentage de la valeur théorique;

M est la valeur de référence, exprimée en pourcentage de la valeur théorique : M=98.5 si $X_m < 98.5$; M=$X_m$ si $98.5 \leq X_m \leq 101.5$; M=101.5 si $X_m > 101.5$;

k est la constante d'acceptabilité (k=2.4 pour 10 sachets);

s est l'écart type des teneurs $X_i$.

| Paramètres d'uniformité de teneur (diosmine) | Lot LP02 1000 litres (sachet) selon l'Exemple 1 | |
|---|---|---|
| | Début ensachage | Fin ensachage |
| Teneur moyenne | 100.5 | 102.1 |
| Ecart-type | 0.9 | 0.7 |
| Valeur d'acceptation (AV) | 2.2 | 2.3 |

Selon la Pharmacopée Européenne, article 2.9.40, une valeur d'acceptation inférieure à 15 signifie que l'uniformité de teneur est conforme (niveau L1). Le tableau ci-dessus montre donc que la diosmine contenue dans la formulation selon l'invention a une uniformité de teneur qui répond aux exigences réglementaires.

**Revendications**

1. Composition pharmaceutique sous la forme d'une suspension orale buvable en sachet, comprenant comme principe actif de la diosmine et optionnellement des dérivés flavonoïques et comme excipient de la gomme xanthane.

2. Composition pharmaceutique selon la revendication 1, dans laquelle le principe actif est une fraction flavonoïque purifiée et micronisée.

3. Composition pharmaceutique selon la revendication 2, dans laquelle la fraction flavonoïque comprend de la diosmine, de l'hespéridine, de l'isorhoifoline, de la linarine et de la diosmétine.

4. Composition pharmaceutique selon la revendication 2, dans laquelle la quantité de la fraction flavonoïque est comprise entre 7%m/m et 20%m/m.

5. Composition pharmaceutique selon la revendication 1, dans laquelle la concentration de gomme xanthane est de 0.5% m/v.

6. Composition pharmaceutique selon la revendication 1, dans laquelle la quantité de gomme xanthane est comprise entre 0.30%m/m et 0.60%m/m.

7. Composition pharmaceutique selon la revendication 1, comprenant un ou plusieurs excipients pharmaceutiquement acceptables choisis parmi les édulcorants, les arômes, les conservateurs ou les correcteurs de pH.

8. Composition pharmaceutique selon l'une quelconque des revendications 1 à 7, pour son utilisation dans le traitement de l'insuffisance veineuse.

**Patentansprüche**

1. Pharmazeutische Zusammensetzung in Form einer oralen, trinkbaren Suspension in einem Beutel, die als Wirkstoff Diosmin und optional Flavonoidderivate und als Hilfsstoff Xanthan enthält.

2. Pharmazeutische Zusammensetzung nach Anspruch 1, wobei der Wirkstoff eine gereinigte und mikronisierte Flavonoidfraktion ist.

3. Pharmazeutische Zusammensetzung nach Anspruch 2, wobei die Flavonoidfraktion Diosmin, Hesperidin, Isorhoifolin, Linarin und Diosmetin umfasst.

4. Pharmazeutische Zusammensetzung nach Anspruch 2, wobei die Menge der Flavonoidfraktion zwischen 7%m/m und 20%m/m liegt.

5. Pharmazeutische Zusammensetzung nach Anspruch 1, wobei die Konzentration von Xanthan 0,5 % w/v beträgt.

6. Pharmazeutische Zusammensetzung nach Anspruch 1, wobei die Menge an Xanthan zwischen 0,30%m/m und 0,60%m/m liegt.

7. Pharmazeutische Zusammensetzung nach Anspruch 1, umfassend einen oder mehrere pharmazeutisch akzeptable Hilfsstoffe, ausgewählt aus Süßungsmitteln, Aromastoffen, Konservierungsmitteln oder pH-Korrekturmitteln.

8. Pharmazeutische Zusammensetzung nach einem der Ansprüche 1 bis 7 zur Verwendung bei der Behandlung von Veneninsuffizienz.

**Claims**

1. Pharmaceutical composition in the form of an oral drinkable suspension in a sachet, comprising as active ingredient diosmin, and optionally flavonoid compounds, and as excipient xanthan gum.

2. Pharmaceutical composition according to claim 1, wherein the active ingredient is a purified and micronised flavonoid fraction.

3. Pharmaceutical composition according to claim 2, wherein the flavonoid fraction comprises diosmin, hesperidin, isorhoifolin, linarin and diosmetin.

4. Pharmaceutical composition according to claim 2, wherein the amount of the flavonoid fraction is between 7% w/w and 20% w/w, inclusive.

5. Pharmaceutical composition according to claim 1, wherein the concentration of xanthan gum is 0.5% w/v.

6. Pharmaceutical composition according to claim 1, wherein the amount of xanthan gum is between 0.30% w/w and 0.60% w/w, inclusive.

7. Pharmaceutical composition according to claim 1, comprising one or more pharmaceutically acceptable excipients selected from sweeteners, aromas, preservatives and pH-adjusters.

8. Pharmaceutical composition according to any one of claims 1 to 7, for use in the treatment of venous insufficiency.

## RÉFÉRENCES CITÉES DANS LA DESCRIPTION

**Documents brevets cités dans la description**

- EP 0711560 A
- US 5240732 A
- US 20120070475 A
- FR 2661610 A1
- FR 2781156 A1
- FR 6967 M